# EUROPEAN PATENT SPECIFICATION

(11) **EP 2 229 970 B1**
(45) Date of publication and mention of the grant of the patent: **06.06.2012**
(21) Application number: 09155216.6
(22) Date of filing: 16.03.2009
(51) Int. Cl.: A61M 5/36, A61M 5/38

(54) **Bubble trap system for an infusion pump device**
Blasenfangsystem für eine Infusionspumpenvorrichtung
Système de débulleur pour un dispositif de pompe à perfusion

(43) Date of publication of application: 22.09.2010
(73) Proprietor: F. Hoffmann-La Roche AG, 4070 Basel (CH); Roche Diagnostics GmbH, 68305 Mannheim (DE)
(72) Inventor: Huwiler, Christoph, 6340, Baar (CH); Kühni, Florian, 3400, Burgdorf (CH); Geipel, Andreas, 4665, Oftringen (CH)
(74) Representative: Rentsch Partner AG

(56) References cited:
- US-A- 3 007 334
- US-A- 4 177 149
- US-A- 4 395 260

## Description

### Field of the Invention

The invention relates to a bubble trap for removing bubbles from a stream of liquid, a container for storing a liquid medicament with such a bubble trap, and a device for the automated release of a liquid medicament, comprising and/or incorporating such a bubble trap and/or such a flexible container, according to the preambles of the independent claims.

### State of the art

Devices for the automated release of liquid medicaments are normally used with patients who have a continuous and in the course of the day varying need of a medicine that can be administered by subcutaneous infusion. Specific applications are, for example, certain pain therapies and the treatment of diabetes, in which computer controlled infusion pump devices, such as insulin pumps, are used. Such devices can be carried by a patient on the body, and contain a certain amount of liquid medicament in a medicine reservoir in the form of a container. The medicine reservoir often comprises medicine sufficient for one or several days. The liquid medicament is supplied to the patient's body from the medicine reservoir through an infusion cannula or an injection needle.

Particularly in self-administration of medicaments, for example insulin, the patients using the medicament in question and administering it themselves by means of an infusion pump device are increasingly emphasizing convenience and discretion. As a consequence the dimensions of such infusion devices are limited, and particularly the overall length, width and thickness should be as small as possible, in order not be evident through clothing and to be carried as comfortable as possible.

While there are fully or partly disposable single-use infusion pump devices, such devices are typically non-disposable and are loaded with a disposable drug cartridge. Such disposable cartridges are preferable for sterility and contamination prevention reasons. They may be delivered pre-filled with a certain liquid medicament, or empty, ready to be filled by a user.

One common type of infusion pump device that is carried on or near the body has a medicine reservoir with a cylindrical ampoule and a displacement piston, which is pushed into the ampoule by a piston rod or threaded spindle in order to convey the liquid medicament. These known designs have the disadvantage of being longer and/or thicker than desired, the resulting dimensions being detrimental to the provision of compact infusion pumps.

In another advantageous type of infusion pump device the rigid container and movable piston of a syringe-type infusion pump are replaced by a flexible container. Such a container may for example have the form of two flexible wall sheets that are sealed together. The liquid medicament is obtained from the container by a downstream pump. Flexible containers have the advantage of a smaller volume surplus of the container in relation to its content, which reduces the manufacture costs and the achievable dimensions of an infusion pump device using such a flexible container. The volume of a flexible container for use in an infusion pump device may be up to 10 ml, but is preferably 5 ml or less, and more preferably lies in a range of 1.5 to 3.5 ml.

A known problem of infusion devices are air bubbles in the fluidic system, particularly in the pump system, but also in other components such as the container or a filling port. It cannot be avoided that at least a certain volume of air is present in a container. The removal of said air requires a certain skill of a user, and in any case cannot be removed completely. If said air remains in the container or in another part of the fluidic system, it may be administered instead of the liquid medicament, which leads to potentially dangerous dosing errors. Furthermore the administration of air into a patient's body should be generally avoided for medical reasons.

Yet another problem of air in the fluidic system is the reduced stiffness of the fluidic system. Due to the high compressibility of gases such as air in relation to liquids such as water, it becomes difficult to measure the exact pressure in the fluidic system. This impedes the detection of blockages or occlusions in the fluidic system by measuring the fluidic pressure.

US 4177149 discloses an air bubble filter device for intravenous liquid administration. A filter chamber is in fluid flow communication with an inlet and an outlet. A filter in the form of a microporous hydrophilic nylon sheet is disposed in the filter chamber across the line of fluid flow through the chamber from the inlet member to the outlet member. A vent in the filter chamber upstream of the filter is covered by a liquid-repellent gas-permeable PTFE membrane. The pores of the filter are saturated by the aqueous liquid, so that the liquid but no gas can penetrate the hydrophilic filter. Gas, but no liquid can pass through the hydrophobic membrane.

US 4395260 shows a drip chamber for intravenous liquid administration with an air bubble filter. A microporous filter is arranged in a filter chamber across the downstream outlet of the filter chamber and precludes particulate matter and larger air bubbles from entering the downstream fluid delivery system A baffle deflects falling drops to the walls of the drip chamber and avoids the formation of micro-bubbles. The filter is arranged on a carrier than can be temporarily lifted, so that air bubbles caught downstream under the filter can escape upstream.

### Objects of the invention

One object of the invention is to provide an advantageous bubble trap for removing bubbles from a stream of liquid, particularly in an infusion pump device or a container for storing a liquid medicament. Particularly a bubble trap according to the invention should be able to retain bubbles exceeding a certain minimum size. Preferably the effectiveness of a bubble trap according to the invention should be independent of the orientation of the bubble trap in the three-dimensional space.

In addition a bubble trap according to the invention should be producible with high quality at low costs, and should comprise a minimum number of components.

Another object of the invention is to provide a flexible container for storing a liquid medicament comprising and/or incorporating such a bubble trap.

Yet another object of the invention is to provide a device for the automated release of a liquid medicament, comprising and/or incorporating such a bubble trap, and/or such a flexible container.

These and other objects are achieved by a bubble trap, a container for storing a liquid medicament, and an infusion pump device, according to the independent claims. Advantageous embodiments are given in the dependent claims.

### Summary of the invention

In a bubble trap according to the invention for removing bubbles from a stream of liquid, a grate is arranged in the stream, adapted to retain bubbles that are drifting in the stream. The grate comprises a grate wall and two or more inlets arranged on the grate wall with a certain, defined geometry, through which the stream can pass downstream. The shape of the inlets and/or the distribution of the inlets on the grate is designed such that a bubble larger than a certain minimum size cannot pass the grate without coming into contact with an inlet.

When an air bubble arrives at an inlet of the bubble trap, it will thus inevitably come into contact with the surface of the grate wall and the inlet. When the air bubble enters the inlet, the contact area between grate and bubble will increase, and the contact area between air bubble and liquid, as well as liquid and grate surface, will decrease. Due to the specific relations of the interface tensions between the gas mixture in the bubble, the grate surface and the liquid, such a state will have a higher potential energy compared to a state with the bubble not entering the inlet. Since the stream of liquid can pass through the other inlets of the grate, however, the necessary pressure difference across the grate will not increase to a value that is sufficient to force the bubble through the inlet. It thus will be retained in front of the inlet. The effect of the interface tension increases with decreasing dimensions of the inlets.

Thus as an effect of the two or more inlets arranged on the grate wall, the liquid flow in the bubble trap according to the invention cannot be blocked by one single air bubble. Preferably the shape and distribution of the arrangement of the two or more inlets on the grate wall is designed such that a bubble below a certain maximum size that is retained by the grate can block only a part of the combined cross-sectional area of the inlets.

In a preferred embodiment of a bubble trap according to the invention the material of the grate is chosen such that the energy related to the entry of a bubble in an inlet is as high as possible. This can be achieved by a grate material that is as hydrophilic as possible. Hydrophilicity of a surface correlates with the energy that has to be expended to separate water from the surface, and consequently with the interface tension between aqueous liquid and grate surface.

For the same reason it is advantageous if the shape of the at least one inlet is designed such that the relation of the surface of the inlet to its cross-sectional area is as large as possible. This can be achieved by choosing the shape of an inlet such that the relation of the circumference of the inlet (which is proportional to the inner surface of the inlet) to its cross-sectional area is larger than for an inlet with circular shape.

Advantageously the grate of the bubble trap, particularly the circumference of the inlets, is sharp-edged. This has the effect that the entry of a bubble into an inlet would lead to a steep increase of the potential energy due to the interface tension, while for soft, rounded edges the increase would be continuously. The latter would increase the probability of a bubble to enter the inlet.

Advantageously a containment chamber is arranged upstream of the grate. The bubble trap may furthermore comprise two or more grates.

In another preferred embodiment the bubble trap according to the invention comprises 2 to 10 inlets, which are arranged on the grate wall with a certain distance to each other.

In yet another embodiment of bubble trap according to the invention, the bubble trap includes from upstream to downstream, in sequence: a supply conduit, a containment chamber, two or more separate conduits leading from an inlet to a common junction point, and an outlet conduit.

To increase the capacity and efficiency of a bubble trap according to the invention, a containment chamber and a second grate can arranged downstream of a first grate.

A bubble trap may be embodied in a flexible container for storing a liquid medicament. In such an especially preferred embodiment, which does not need additional parts or elements, the flexible container consists of two wall sheets of flexible material that are sealed together, and with an insert part arranged between the two wall sheets with positive locking. The insert part comprises an essentially flat body with an inner conduit opening toward an upper surface of the body. The inner conduit is fluidly connected to two or more tubular conduits that lead to an outer edge of the body, and open to a special containment chamber, or preferably to a storage compartment of the flexible container.

In another especially preferred embodiment of a bubble trap according to the invention, the bubble trap is embodied in a container for storing a liquid medicament, comprising a port attached to a wall of the container. The port comprises a base plate having two or more tubular conduits that lead to an outer edge of the base plate. Said tubular conduits open to a special containment chamber, or preferably to a storage compartment of the flexible container.

In yet another especially preferred embodiment of a bubble trap according to the invention, the bubble trap is embodied in a flexible container for storing a liquid medicament, with a wall consisting of two sheets of flexible material that are sealed together, and wherein two or more drain channels are arranged between a storage volume and an access opening of the container. The two drain channels are formed by a cavity arranged on one or both of the two wall sheets, and open to a special containment chamber, or preferably to a storage compartment of the flexible container.

In another embodiment of a bubble trap according to the invention, a membrane is arranged in contact with a compartment directly upstream of the grate. The membrane is adapted to let permeate gases, while retaining the liquid in the compartment. This will allow bubbles retained in the bubble trap to leave the compartment, hereby increasing the capacity of the bubble trap. An embodiment with such a degassing membrane is adapted for systems with overpressure, which is for example the case for syringe type dosing pumps, since the pressure difference to the space behind the membrane will be the driving force for the air in the bubbles to pass the membrane.

An advantageous container for storing a liquid medicament according to the invention comprises a bubble trap according to the invention, while an advantageous device for the automated release of a liquid medicament, particularly an infusion pump device, comprises, incorporates or is capable of using a bubble trap according to the invention and/or a flexible container according to the invention.

In a method according to the invention for degassing a liquid, particularly a liquid medicament, a stream of liquid is directed through a bubble trap according to the invention as described above.

Prior to being put into operation a bubble trap according to the invention has to be filled with liquid. Care has to be taken that no air bubbles are already initially present in the bubble trap, particularly downstream of the grate. This can be achieved by filling the bubble trap upstream, supplying the liquid in reverse direction. Thus in a method according to the invention for filling a bubble trap an outlet conduit of the bubble trap is connected to a pressurized or non-pressurized liquid supply, for example a vial, and the bubble trap is filled with liquid in reverse flow direction, from the outlet conduit through the inlets of the grate of the bubble trap. Preferably the bubble trap is evacuated prior to this reversed filling. Advantageously the bubble trap is furthermore connected upstream to a flexible container, which then is also evacuated and subsequently filled with liquid, along with the bubble trap.

As used herein, the term "air" is meant to encompass any gas or mixture of gases forming stable bubbles in a liquid. The terms "medicament" and "liquid medicament" are meant to encompass any drug-containing flowable medicine, or therapeutic or diagnostic liquid, capable of being passed through a delivery element such as a hollow needle in a controlled manner, such as a liquid, solution, gel or fine suspension. Representative drugs include pharmaceuticals such as peptides, proteins, and hormones, biologically derived or active agents, hormonal and gene based agents, nutritional formulas and other substances in both solid (dispensed) or liquid form. In particular the term medicament encompasses insulin preparations ready for administration. The terms "subcutaneous infusion" and "subcutaneous injection" are meant to encompass any method in which a needle device is inserted at a selected site within the body of a patient for subcutaneous, intravenous, intramuscular or intradermal delivery of a liquid medicament to a subject. Further, the term needle defines a piercing member (including an array of micro needles) adapted to be introduced into or through the skin of a subject.

### Brief description of the drawings

In order to facilitate a fuller understanding of the present invention, reference is now made to the appended drawings. These references should not be construed as limiting the present invention, but are intended to be exemplary only.
- Figure 1: schematically shows a cross-section of an embodiment of a bubble trap according to the invention.
- Figure 2: schematically shows a cross-section of another embodiment of a bubble trap according to the invention, with two containment cham- bers.
- Figure 3: schematically shows a further embodiment of a bubble trap, (a) in a top view with the cover removed, and (b) in a cross section.
- Figure 4: schematically shows a cross-section of yet another embodiment of a bubble trap according to the invention, with two grates.
- Figure 5: schematically shows a variant of a bubble trap according to the in- vention, embodied in a flexible container with an insert part, with the insert part (a) in a side view, (b) in a cross-section, and (c) in a top view, and (d) with the flexible container with the insert part.
- Figure 6: schematically shows another variant of a bubble trap according to the invention, embodied in a flexible container with a port, with the port (a) in a perspective view, (b) in a top view, and (c) in a cross-sectional view, and (d) with the port mounted on the flexible container.
- Figure 7: schematically shows an embodiment of a bubble trap according to the invention, realized as an integral part of a flexible container.
- Figure 8: schematically shows different variants of inlet fronts in a bubble trap according to the invention.

### Description of embodiments of the invention

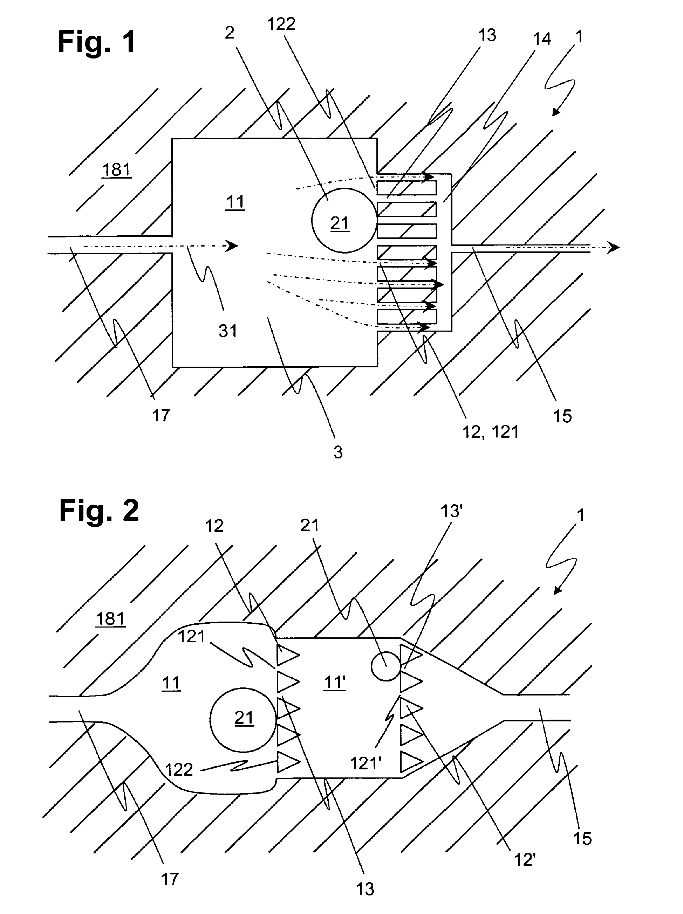

A possible embodiment of a bubble trap 1 according to the invention is schematically shown in Figure 1, in a cross-section through the body of the bubble trap 1. A containment chamber 11 is in fluid connection 17 with a reservoir of liquid and/or a conduit system delivering liquid. In the flow direction (from left to right in Figure 1) a grate 12 is arranged at the end of the containment chamber 11, for retaining air bubbles 21 drifting in the stream of liquid. In the shown embodiment the grate 12 consists of a front wall 122 of the containment chamber 11, on which six inlets 121 are arranged. The conduits 13 subsequent to the six inlets 121 lead to a junction 14, and an outlet conduit 15.

When an air bubble 21 arrives in the containment chamber 11, dragged along with the stream of liquid 31, it will finally move toward an inlet 121 of the grate 12. However, since due to the relation of interface tensions between the air 2, the liquid 3, and the surface of the walls of the bubble trap 1, the necessary pressure difference across the grate 12 that would be necessary to force the bubble 21 through the inlet 121 and conduit 13 is larger than the pressure difference caused by the remaining inlets 121 that are not obstructed by the bubble 21. As a consequence the bubble 21 does not enter the inlet 121, and remains in the containment chamber 11.

A basic requirement is of course that the inlets 121 are equal or smaller than the expected arriving air bubbles. The minimum size of the air bubbles that can be retained by a specific bubble trap 1 according to the invention is thus defined by the maximum diameter of a spherical bubble that can enter an inlet 121 of that specific bubble trap 1 according to the invention. A useful range for the diameter of an inlet of a bubble trap according to the invention is 0.01 to 0.3 mm. Preferably the diameter lies in a range of 0.05 to 0.2 mm. Generally smaller diameters are preferable, as long as manufacturability is given.

Preferably the edges of the inlets 121 are sharp. Chamfered edges could reduce the retaining effect of the bubble trap, since the potential energy due to the interface tension of a bubble entering the inlet increases slower.

The depicted bubble trap 1 will retain air bubbles 2 as long as the total effective cross-section of the remaining open inlets 121 is sufficient to keep the pressure difference across the grate 12, between the containment chamber 11 and the downstream conduit 15, below the threshold where the bubble 21 could flow into the inlet 13. If only two inlets are present, the distance between the two inlets should for example be 5 to 100 times larger then the diameter of the inlet.

In an advantageous embodiment of a bubble trap according to the invention the volume of the containment chamber 11 is thus sufficiently large to contain several bubbles. It is also preferable that the area of the front of the grate 12 is sufficiently large to leave space for several air bubbles. At the same time the volume of the containment chamber should be as small as possible, in order to keep the dead volume of a container or an infusion pump device small. Said dead volume is that part of liquid medicament volume that cannot be used, since it cannot be removed from a container or an infusion pump device. Especially for very expensive liquid medicaments and/or small overall volumes of a container the dead volume can have a considerable detrimental effect on the cost efficiency. Preferably the volume of a non-drainable containment chamber should lie between 20 to 200 µl.

Alternatively to a specific containment chamber 11, the grate 12 of the bubble trap can also be arranged in a supply channel that is sufficiently wide to provide sufficient space for the retained air bubbles.

Figure 2 depicts an advantageous embodiment of a bubble trap 1 according to the invention, in which two containment chambers 11, 11' are arranged in sequence. If for some reason an air bubble 21 passes the first grate 12, it will arrive in a second containment chamber 11', where it is retained. A bubble trap according to the invention may comprise more than two containment chambers and grates, which will further increase the effectiveness and the capacity of the bubble trap, and reduce the probability of a bubble to pass the bubble trap.

A further advantage of the embodiment shown in Figure 2 is caused by the fact that the diameter of the conduits 13, 13' becomes larger with increasing distance from the supply conduit. While for an air bubble 21 drifting downstream arriving at the grate 12 this does not make a difference, since the grate 12 is still sharp-edged, the passage of a bubble 21 through the gate 12 in reverse direction is facilitated. If the shown bubble trap 1 is purposefully degassed, by streaming liquid from downstream to upstream (right to left in Figure 2), or when a container is filled via the bubble trap 1 (reversed filling), an air bubble present in the downstream fluid system will easily pass the grate 12 in the reverse direction.

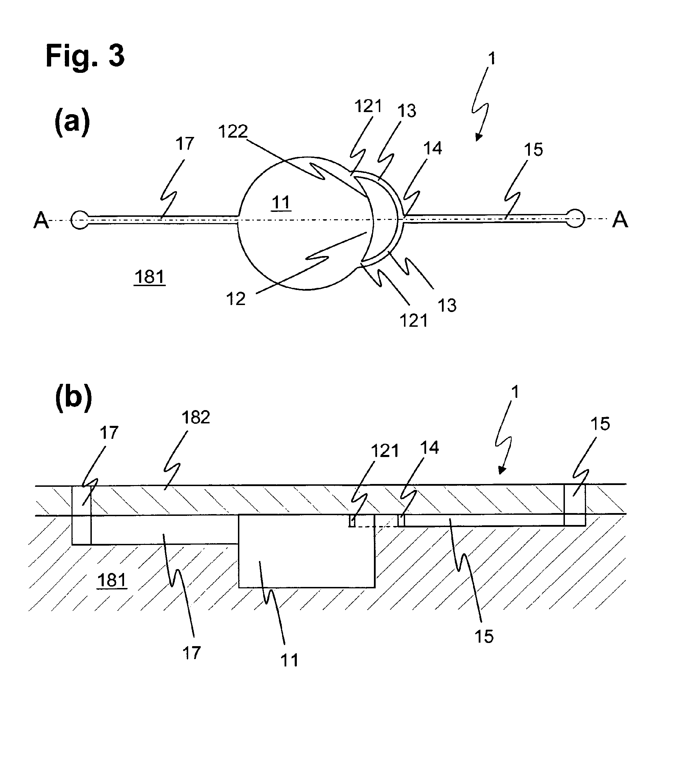

Yet another embodiment of a bubble trap according to the invention is depicted in Figure 3, (a) in a top view with removed cover 182 of the bubble trap 1, and (b) and in a cross-section along line A-A. The shown bubble trap 1 comprises a body 181 and a cover 182. From an inlet of the bubble trap a supply conduit 17 leads to a cylindrical containment chamber 11. Two bent conduits 13 exit from the chamber 11, and are joined 14 to an outlet conduit 15, leading to a bubble trap outlet.

The height of the chamber 11 is larger than the height of the inlets 121, on one hand to provide sufficient space for retained air bubbles, and on the other hand to decrease the cross-section area of the inlets 121 in relation to the total area of the grate wall 122, thereby improving the trap effect. Another advantage of the shown embodiment is that the two Inlets are shifted sideward in relation to the of the supply conduits.

In an advantageous embodiment of such a bubble trap, the walls of the conduits 17, 13, 15 and the chamber 11 are slightly sloped, which allows the use of a simple injection-molding tool for the manufacture of the bubble trap.

The embodiments of bubble traps according to the invention discussed so far are especially useful for the integration into an adapter or connection device, located between a container or reservoir of liquid medicine and the pump device.

It is also possible to integrate a bubble trap 1 according to the system in certain parts or elements of an infusion pump device, and/or a flexible container for a liquid medicament. In the European patent application with the title "Flexible container with insert part" of the applicants, filed on the same day as this present application and published as EP 2229927 A1, an advantageous flexible container for storing a liquid medicament is disclosed. Said application is hereby incorporated by reference as part of this disclosure in its entirety.

The flexible container disclosed in said application comprises a container wall consisting of two wall sheets of flexible material that are sealed together, a storage compartment for the liquid medicament, and an access point on one of the wall sheets. The storage compartment and the access point are in fluid connection. The access point is adapted to be fluidly connected to an outer conduit system, for example of an infusion pump device. For fluidly connecting the storage compartment and the access point, an insert part is arranged between the two wall sheets with positive locking.

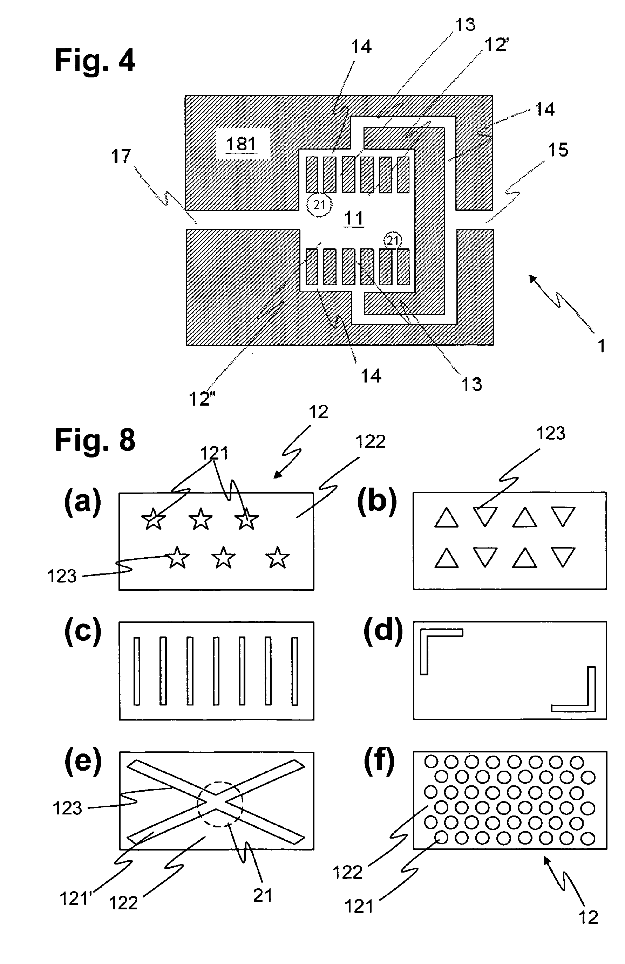

Yet another possible embodiment of a bubble trap 1 according to the invention is shown in Figure 4, with two grates 12', 12" arranged on opposite sides of the containment chamber 11. The conduits 13 of both grates 12', 12" lead to a common junction 14 opening toward the outlet conduit 14. The two sub-grates 12', 12" are thus part of one common grate 12 of the bubble trap 1. This particular embodiment has the additional advantage that depending on the orientation of the bubble trap in space, the bubbles 21 will tend to remain on the sub-grate lying on the upper side, while the other sub-grate remains free of bubbles. Alternatively one may also use three of more sub-grates. Particularly the containment chamber 11 may comprise a multitude of walls that are all equipped with a sub-grate 12', 12".

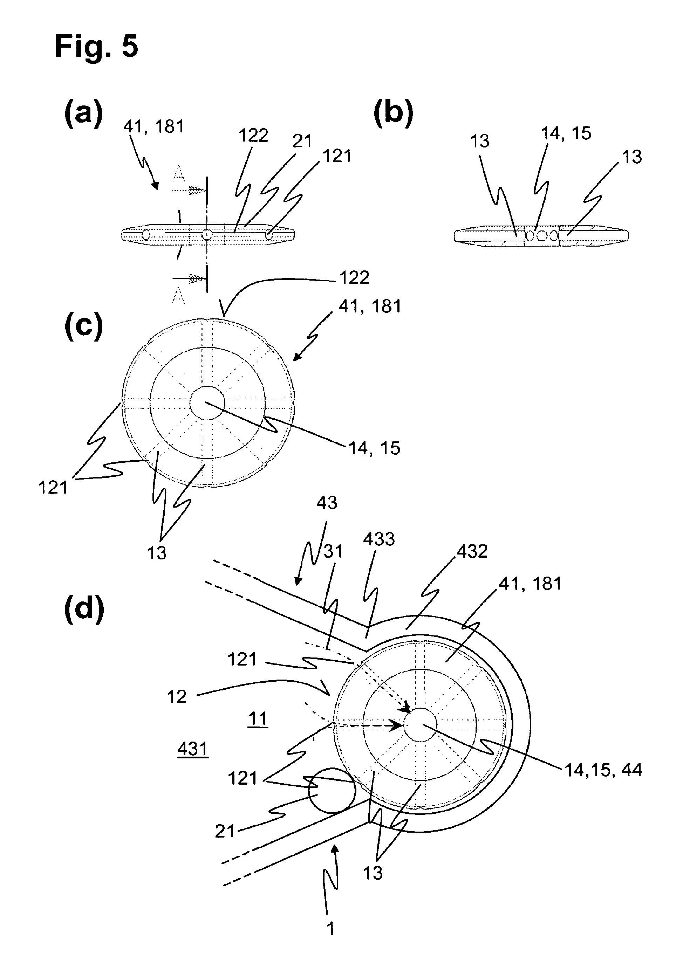

A particularly advantageous embodiment of such an insert part 41 is shown in Figure 5(a) to (c), and a detail of a flexible container 43 with such an insert part 41 is shown in Figure 5(d). The flexible container 43 and the insert 41 are designed in a way that in combination they act as a bubble trap 1 according to this invention. On one end of the flexible container the insert part 41 is arranged between the two wall sheets of the container 43. The sealing rim 432 forms a neck 433 that is smaller than the diameter of the disc like shaped insert part 41, thereby positively locking the insert part within the flexible container.

The insert part 41 of the flexible container has an essentially disc shaped form, with a bore 15 arranged in the center of the disc, leading to an access point 44 in the wall of the container. Eight tubular conduits 13 are radially arranged inside the body 181 of the insert part 41. Due to the highly symmetric form of the insert part, the angular orientation of the insert part in relation to the flexible container is irrelevant. In every orientation angle at least one conduit 13 will open to the storage compartment 431 of the container, providing a fluid connection 13, 14, 15 between the access point 44 and the storage compartment 431.

The container 43 and the insert part 41 form a bubble trap 1 according to the invention. The openings of the conduits 13 that face toward the inner storage compartment 431 act as the inlets 121 of the grate 12 of the bubble trap 1. An air bubble 21 arriving at the insert part 41 cannot enter a conduit 13, and thus will be retained inside the storage compartment 431 of the container 43.

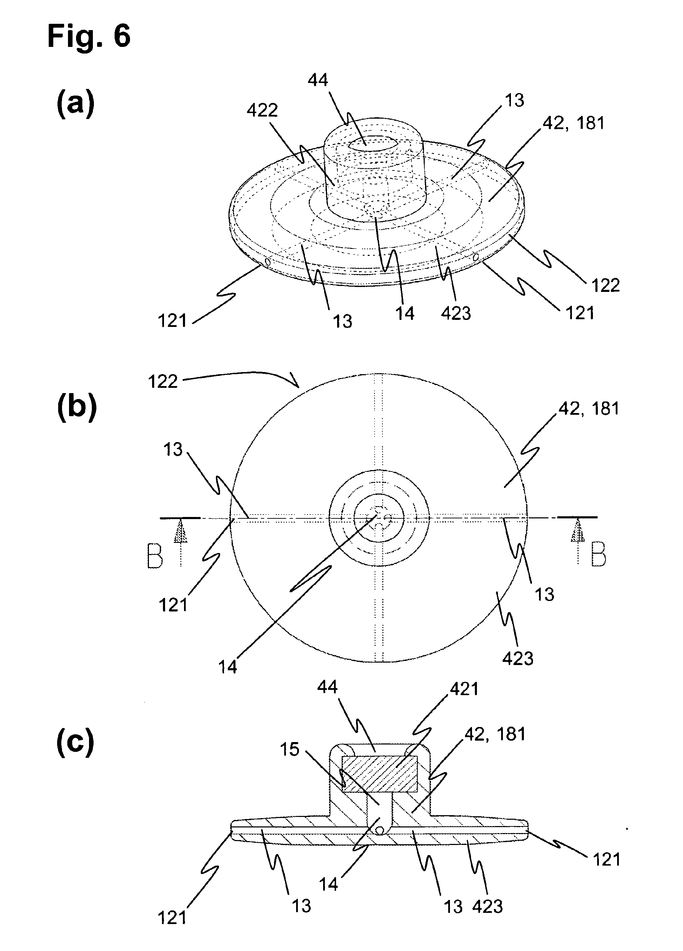

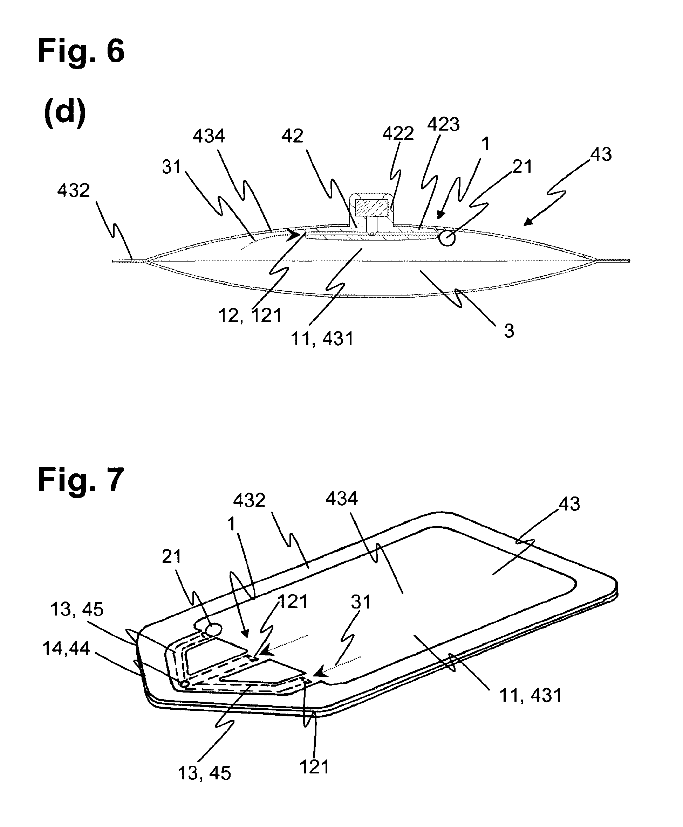

Another example of a bubble trap 1 according to the invention formed in a flexible container is depicted in Figure 6. Flexible containers may be provided with one or more ports mounted to the container wall, in addition to the access area. These ports may be used for transferring liquid to and from the storage compartment of the container, or may be used to deaerate the container. A particularly advantageous form of such a port is disclosed in European Patent Application No. 08167548 of the applicants, which is hereby incorporated by reference as part of this disclosure in its entirety. Said application teaches a flexible container for storing a liquid medicament, comprising two walls consisting of a flexible, sheet-like material, and a port mounted to the wall for transferring liquid to and from a storage compartment of the container. The port has a flange that is sealingly attached to the wall, and an inner conduit connecting the storage compartment and the exterior of the container. The port comprises a base plate facing toward the storage compartment, and having at least one drain channel arranged on said base plate. The drain channels are connected to the inner conduit via an inner opening located on the base plate.

Ports as they are disclosed in that application can be modified, in order to act as a bubble trap 1 according to the invention, and without affecting the other advantageous effects of the ports. An example of such a modified port 42 is disclosed in Figure 6. The port 42 shown in Figure 6(a) to (c) comprises an adapter 422 arranged on a base plate 423. When mounted on a flexible container 43 (Figure 6(d)), the adapter 422 protrudes through a circular hole in the container wall 434. The surface of the base plate 423 on the same side as the adapter forms the flange for mounting the port 42 to the wall 434. Said flange is sealed in a liquid tight manner to the inner side of the wall, for example by ultrasonic welding or gluing.

Four tubular conduits 13 are arranged in the in the base plate 423, leading from the peripheral edge 122 of the base plate to a central junction 14 in liquid connection to an outlet conduit 15. In the shown embodiment a septum 431, for example made from silicon polymer, is arranged in the outlet conduit 15. This particular embodiment is thus suitable for the use of a hollow needle penetrating the septum 431 to connect the storage compartment 11 of the container 43 to an outer conduit system, and to transfer a liquid medicament into and out of the flexible container 43.

When mounted on a flexible container 42, as shown in Figure 6(d), the port 42 in combination with the container 43 forms a bubble trap 1 according to the invention. The four openings of the conduits 13 facing toward the inner storage compartment 431 act as the inlets 121 of the grate 12 of the bubble trap 1. The containment chamber 11 of the bubble trap is provided by the storage compartment 431. An air bubble 21 in the container arriving at the port 42 cannot enter the conduits 13, and thus will be retained inside the storage compartment 431 of the container 43.

Yet another example of a bubble trap 1 according to the invention embodied in a flexible container is shown in Figure 7. The disclosed flexible container is a modification of an advantageous flexible container for storing a liquid medicament as they are disclosed in the European Patent Application No. 08170627 of the applicants. The content of said application is hereby incorporated by reference as part of this disclosure in its entirety. These flexible containers comprise a wall consisting of two sheets of flexible material that are sealed together, a storage compartment intended for the liquid medicament, and an access opening in fluid connection with the storage compartment, intended to be fluidly connected to an external conduit, for example of an infusion pump device. A drain channel is arranged between the storage compartment and the access opening, the drain channel being formed by a cavity on one or both of the two wall sheets. Preferably the cavity is located between an oblong corrugation or groove in one of the wall sheets and the opposite sheet.

Such a flexible container as described above can be modified, in order to integrate a bubble trap according to the invention. An example of such a flexible container with a bubble trap 1 according to the invention is shown in Figure 7, in its empty state. The flexible container 43 basically consists of two wall sheets 434 that are sealed along a peripheral sealing rim 432, and form a storage compartment 431. On one end of the container three drain channels 45 are arranged in a broadened area of the sealing rim 432, formed by oblong depressions in one of the wall sheets. The drain channels 45 are connected at a common access opening 44, and lead to the storage compartment 431. In contrast to the flexible containers disclosed in European Patent Application No. 08170627 of the applicants, where only one drain channel connects the access opening and the storage compartment, the multitude of narrow drain channels 45 opening to the storage compartment 431 act as a grate 12 of a bubble trap 1 according to the invention. When an air bubble arrives at an inlet 121 of one of the drain channels 45, which are equivalent to the conduits 13 of the bubble trap, it will be retained and remains in the containment chamber 11 respectively storage compartment 431.

In the embodiments of bubble traps according to the invention discussed so far the inlets 121 had basically a circular or elliptical shape. Such a design, however, does not have to be the most ideal solution. Since the mechanism of the bubble trap is based on the fact that there is a considerably higher interface tension between the air of the bubble and the surface of the bubble trap body than between the liquid and the surface of the bubble trap, and the energy related to said interface tension is proportional to the interface area, it is preferable that the potential interface area between an entering bubble 21 and the inlet 121 and conduit 13 is as large as possible, in order to increase the bubble trap effect. Thus one may adapt the shape on-an inlet and/or the conduit to increase the interface area.

Figure 8 schematically shows different variants of inlets 121 of a bubble trap according to the invention, and their distribution on the grate 12. For example the inlets may have a star like shape as in Figure 8(a), or a triangular shape as in Figure 8(b). These two variants have the advantage that the surface of the bubble trap body that will be in contact with the air of a bubble of a given volume will be larger than for an inlet having a circular shape with the same cross-sectional area. Figure 8(c) shows an embodiment with inlets in the form of parallel slots, and in Figure 8(d) two chevron-like inlets 121 are arranged.

In Figure 8(e) a variant of a bubble trap according to the invention is depicted in which only one single cross-like shaped inlet 121 is arranged. Although this embodiment comprises actually only one inlet 121 and conduit 13, and the overall cross-sectional area of the inlet 121 may be even larger than the cross-section of the air bubble 21 to be retained (symbolized by the dashed circle), the local cross-sectional area of the inlet across the dimensions of a bubble will be smaller, and consequently the bubble is retained. The one single inlet 121' thus in effect acts as a multitude of distinct inlets 121, and consequently has to be understood as "two or more inlets 121" in the sense of the independent claim on the bubble trap. Figure 8(d) depicts a further variant, in which the density of inlets 121 is higher than in the previously discussed examples. The resulting grate 12 is essentially a sieve.

One particular advantage of the embodiments of bubble traps according to the invention as shown in Figure 8(f) is the fact that the inlets 121 are arranged on the grate 12 in a way that ensures that for every conceivable orientation of an air bubble there will be at least one open inlet 121 that is not blocked by the bubble 21. Of course theoretically all inlets might be blocked by a multitude of bubbles. However, depending on the current orientation of the bubble trap in the three-dimensional space, the retained bubbles will tend to move upwards, leaving the inlets located downwards free. Furthermore due to interface tension neighboring bubbles tend to join, forming one single bubble that blocks a smaller area of the grate.

### List of Reference Numerals

- 1: bubble trap
- 11, 11': containment chamber
- 12, 12', 12": grate
- 121, 121': inlet
- 122: grate wall
- 123: rim of inlet
- 13, 13': conduit
- 14: junction
- 15: outlet conduit
- 17: supply conduit
- 181: body of the bubble trap
- 182: cover of the bubble trap
- 2: air, or any other gas
- 21: bubble
- 3: liquid
- 31: stream of liquid
- 41: insert part
- 42: port
- 421: septum
- 422: adapter
- 423: base plate
- 43: flexible container
- 431: storage compartment
- 432: sealing rim
- 433: neck
- 434: wall
- 44: access opening
- 45: drain channels

## Claims

1. A bubble trap (1) for removing bubbles (21) from a stream (31) of liquid (3), with a grate (12) arranged in the stream (31), adapted to retain bubbles (21) that are drifting with the stream (31), **characterized in that** the grate (12) comprises a grate wall (122) and two or more inlets (121) arranged on the grate wall (122) with a certain, defined geometry, through which the stream (31) can pass downstream (15), wherein the shape of the inlets (121) and/or the distribution of the inlets (121) on the grate (1 2) is designed such that a bubble (21) larger than a certain minimum size cannot pass the grate (12) without coming into contact with an inlet (121), and wherein the circumference of the inlets (121) is sharp-edged.

2. The bubble trap according to claim 1, **characterized in that** the shape and distribution of the arrangement of inlets (121) on the grate (12) is designed such that a bubble (21) below a certain maximum size that is retained by the grate (12) can block only a part of the combined cross-sectional area of the inlets (121).

3. The bubble trap according to claim 1 or 2, **characterized by** a containment chamber (11) arranged upstream of the grate (12).

4. The bubble trap according to any of the preceding claims, **characterized in that** the bubble trap comprises two to ten inlets (121) that are arranged on the grate wall (122) with a certain distance to each other.

5. The bubble trap according to any of the preceding claims, **characterized in that** the surface of the grate (12) is hydrophilic.

6. The bubble trap according to any of the preceding claims, **characterized in that** the bubble trap (1), from upstream to downstream, in sequence includes: a supply conduit (17), a containment chamber (11), two or more separate conduits (13) leading from an inlet (121) to a common junction point (14), and an outlet conduit (15).

7. The bubble trap according to any of the preceding claims, **characterized in that** a containment chamber (11') and a second grate (12') is arranged downstream of a first grate (12).

8. The bubble trap according to any of the preceding claims, **characterized in that** the shape of the inlets (121) is designed such that the relation of the circumference of an inlet (121) to its cross-sectional area is larger than for an inlet with circular shape.

9. The bubble trap according to any of the preceding claims, **characterized in that** the bubble trap (1) comprises two or more grates (12', 12").

10. The bubble trap according to any of the preceding claims, **characterized by** a membrane arranged in contact with a compartment (11) directly upstream of the grate (12), wherein the membrane is adapted to let permeate gases.

11. A container (43) for storing a liquid medicament, with a bubble trap (1) according to any of claims 1 to 10.

12. A device for the automated release of a liquid medicament, particularly an infusion pump device, comprising or incorporating a bubble trap (1) according to any of claims 1 to 10, and/or comprising or incorporating a container according to claim 11.

13. A method for degassing a liquid (3), particularly a liquid medicament, wherein a stream of liquid (31) is directed through a bubble trap (1) according to any of claims 1 to 10.

14. A method for filling a bubble trap (1) according to any of claims 1 to 10, wherein an outlet conduit (15) of the bubble trap (1) is connected to a pressurized or non-pressurized liquid (3) supply, and the bubble trap (1) is filled with liquid (3) in reverse flow direction, from the outlet conduit (15) through the inlets (121) of the grate (12) of the bubble trap (1).

## Patentansprüche

1. Blasenfalle (1) zum Entfernen von Blasen (21) aus einem Strom (31) von Flüssigkeit (3), mit einem im Strom (31) angeordneten Gitter (12), das geeignet ist, mit dem Strom (31) treibende Blasen (21) zurückzuhalten, **dadurch gekennzeichnet, dass** das Gitter (12) eine Gitterwand (122) und zwei oder mehr Einlässe (121) umfasst, die mit einer bestimmten definierten Geometrie auf der Gitterwand (122) angeordnet sind und durch die der Strom (31) stromabwärts (1 5) passieren kann, wobei die Form der Einlässe (121) und/oder die Verteilung der Einlässe (121) auf dem Gitter (12) so ausgelegt sind, dass eine Blase (21), deren Größe über einer bestimmten Mindestgröße liegt, nicht durch das Gitter (12) passieren kann, ohne mit einem Einlass (121) in Kontakt zu kommen, und wobei der Umfang der Einlässe (121) scharfkantig ist.

2. Blasenfalle nach Anspruch 1, **dadurch gekennzeichnet, dass** die Form und die Verteilung der Anordnung der Einlässe (121) auf dem Gitter (12) so ausgelegt sind, dass eine Blase (21), deren Größe unter einer bestimmten Maximalgröße liegt und die vom Gitter (12) zurückgehalten wird, nur einen Teil der kombinierten Querschnittsfläche der Einlässe (121) blockieren kann.

3. Blasenfalle nach Anspruch 1 oder 2, **gekennzeichnet durch** eine stromaufwärts vom Gitter (12) angeordnete Rückhaltekammer (11).

4. Blasenfalle nach einem der vorhergehenden Ansprüche, **dadurch gekennzeichnet, dass** die Blasenfalle zwei bis zehn Einlässe (121) umfasst, die in einem bestimmten Abstand voneinander auf der Gitterwand (122) angeordnet sind.

5. Blasenfalle nach einem der vorhergehenden Ansprüche, **dadurch gekennzeichnet, dass** die Oberfläche des Gitters (12) hydrophil ist.

6. Blasenfalle nach einem der vorhergehenden Ansprüche, **dadurch gekennzeichnet, dass** die Blasenfalle (1) von stromaufwärts nach stromabwärts nacheinander Folgendes aufweist: eine Zufuhrleitung (17), eine Rückhaltekammer (11), zwei oder mehr getrennte Leitungen (13), die von einem Einlass (121) zu einer gemeinsamen Verbindungsstelle (14) führen, und eine Auslassleitung (15),

7. Blasenfalle nach einem der vorhergehenden Ansprüche, **dadurch gekennzeichnet, dass** stromabwärts von einem ersten Gitter (12) eine Rückhaltekammer (11') und ein zweites Gitter (12') angeordnet sind.

8. Blasenfalle nach einem der vorhergehenden Ansprüche, **dadurch gekennzeichnet, dass** die Form der Einlässe (121) so ausgelegt ist, dass das Verhältnis des Umfangs eines Einlasses (121) zu seiner Querschnittsfläche größer ist als für einen Einlass mit Kreisform.

9. Blasenfalle nach einem der vorhergehenden Ansprüche, **dadurch gekennzeichnet, dass** die Blasenfalle (1) zwei oder mehr Gitter (12', 12") umfasst.

10. Blasenfalle nach einem der vorhergehenden Ansprüche, **gekennzeichnet durch** eine Membran, die in Kontakt mit einer direkt stromaufwärts vom Gitter (1 2) liegenden Kammer (11) angeordnet ist, wobei die Membran dazu eingerichtet ist, Gase durchdringen zu lassen.

11. Behälter (43) zur Lagerung eines flüssigen Arzneimittels, mit einer Blasenfalle (1) nach einem der Ansprüche 1 bis 10.

12. Vorrichtung zur automatischen Abgabe eines flüssigen Arzneimittels, insbesondere eine Infusionspumpenvorrichtung, die eine Blasenfalle (1) nach einem der Ansprüche 1 bis 10 umfasst oder in die eine Blasenfalle (1) nach einem der Ansprüche 1 bis 10 integriert ist und/oder die einen Behälter nach Anspruch 11 umfasst oder in die ein Behälter nach Anspruch 11 integriert ist.

13. Verfahren zum Entgasen einer Flüssigkeit (3), insbesondere eines flüssigen Arzneimittels, wobei ein Flüssigkeitsstrom (31) durch eine Blasenfalle (1) nach einem der Ansprüche 1 bis 10 geleitet wird.

14. Verfahren zum Füllen einer Blasenfalle (1) nach einem der Ansprüche 1 bis 10, wobei eine Auslassleitung (15) der Blasenfalle (1) mit einem unter Druck stehenden oder nicht unter Druck stehenden Vorrat einer Flüssigkeit (3) verbunden ist, und die Blasenfalle (1) in umgekehrter Strömungsrichtung mit Flüssigkeit (3) gefüllt wird, von der Auslassleitung (15) her durch die Einlässe (121) des Gitters (12) der Blasenfalle (1).

## Revendications

1. Débulleur (1) destiné à éliminer les bulles (21) d'un flux (31) de liquide (3), comprenant une grille (12) disposée dans le flux (31), prévue pour retenir des bulles (21) qui sont emportées par le flux (31), **caractérisé en ce que** la grille (12) comprend une paroi de grille (122) et deux entrées ou plus (121) agencées sur la paroi de grille (122) suivant une certaine géométrie définie, à travers lesquelles le flux (31) peut s'écouler en aval (15), la forme des entrées (121) et/ou la répartition des entrées (121) sur la grille (12) étant conçues de telle sorte qu'une bulle (21) dépassant une certaine taille minimale ne puisse pas passer à travers la grille (12) sans venir en contact avec une entrée (121), la circonférence des entrées (121) étant à bords vifs.

2. Débulleur selon la revendication 1, **caractérisé en ce que** la forme et la répartition de l'agencement des entrées (121) sur la grille (12) sont conçues de telle sorte qu'une bulle (21) inférieure à une certaine taille maximale qui est retenue par la grille (12) puisse bloquer seulement une partie de la superficie combinée en section transversale des entrées (121).

3. Débulleur selon la revendication 1 ou 2, **caractérisé par** une chambre de confinement (11) agencée en amont de la grille (12).

4. Débulleur selon l'une quelconque des revendications précédentes, **caractérisé en ce que** le débulleur comprend deux à dix entrées (121) qui sont agencées sur la paroi de la grille (122) à une certaine distance les unes des autres.

5. Débulleur selon l'une quelconque des revendications précédentes, **caractérisé en ce que** la surface de la grille (12) est hydrophile.

6. Débulleur selon l'une quelconque des revendications précédentes, **caractérisé en ce que** le débulleur (1), de l'amont vers l'aval, inclut, en succession: un conduit d'alimentation (17), une chambre de confinement (11), deux conduits séparés ou plus (13) menant d'une entrée (121) à un point de jonction commun (14), et un conduit de sortie (15).

7. Débulleur selon l'une quelconque des revendications précédentes, **caractérisé en ce qu'**une chambre de confinement (11') et une deuxième grille (12') sont agencées en aval d'une première grille (12).

8. Débulleur selon l'une quelconque des revendications précédentes, **caractérisé en ce que** la forme des entrées (121) est conçue de telle sorte que la relation entre la circonférence d'une entrée (121) à sa superficie en section transversale soit plus grande que pour une entrée de forme circulaire.

9. Débulleur selon l'une quelconque des revendications précédentes, **caractérisé en ce que** le débulleur (1) comprend deux grilles ou plus (12', 12").

10. Débulleur selon l'une quelconque des revendications précédentes, **caractérisé par** une membrane agencée en contact avec un compartiment (11) directement en amont de la grille (12), la membrane étant prévue pour laisser passer les gaz de perméation.

11. Récipient (43) pour stocker un médicament liquide, comprenant un débulleur (1) selon l'une quelconque des revendications 1 à 10.

12. Dispositif pour la remise automatisée d'un médicament liquide, en particulier un dispositif de pompe à perfusion, comprenant ou incorporant un débulleur (1) selon l'une quelconque des revendications 1 à 10, et/ou comprenant ou incorporant un récipient selon la revendication 11.

13. Procédé pour dégazer un liquide (3), en particulier un médicament liquide, dans lequel un flux de liquide (31) est dirigé à travers un débulleur (1) selon l'une quelconque des revendications 1 à 10.

14. Procédé pour remplir un débulleur (1) selon l'une quelconque des revendications 1 à 10, dans lequel un conduit de sortie (15) du débulleur (1) est relié à une alimentation en liquide pressurisé ou non pressurisé (3), et le débulleur (1) est rempli du liquide (3) dans un sens d'écoulement inverse, depuis le conduit de sortie (15) à travers les entrées (121) de la grille (12) du débulleur (1).
